# EUROPEAN PATENT APPLICATION

(11) **EP 2 009 110 A1**
(43) Date of publication of application: **31.12.2008**
(21) Application number: 07111087.8
(22) Date of filing: 26.06.2007
(51) Int. Cl.: C12Q 1/04, C12Q 1/06, C12Q 1/10

(54) **Rapid enumeration of antimicrobial resistant organisms using the Most Probable Number method**

(71) Applicant: National University of Ireland Galway, Galway (IE)
(72) Inventor: Cormican, Martin, Galway (IE); Morris, Dearbhaile, Galway (IE); Galvin, Sandra, Galway (IE)
(74) Representative: Gates, Marie Christina Esther

(57) **Abstract**

Rapid enumeration of antimicrobial resistant organisms using the Most Probable Number method.

The invention provides processes that can be used to examine the occurrence of antimicrobial resistant organisms in water and effluent, and other liquidised substances such as a suspension, e.g. food, soil, faeces etc. The method is capable of detecting antimicrobial resistant microbes in a number of samples simultaneously.

## Description

### Field of the Invention

The field of the invention relates to testing for bacteria resistant to antimicrobial agents such as but not limited to antibiotics. In particular, the invention relates to a method of enumeration of antimicrobial resistant organisms for use in a variety of industries including the environmental, food and medical sectors.

### Background to the Invention

Antimicrobial resistance is a major public health problem, resulting in significant health care costs and mortality rates worldwide. The contribution of the environment to the occurrence and dissemination of antimicrobial resistance is of particular interest in the field of environmental testing.

A number of established testing methods are currently employed for the detection of microorganisms in waters. Firstly, it is common to enumerate *E*. *coli* and bacteria by a membrane filtration technique. Bacterial enumeration is effected through use of the filter method in duplicate. The sample is passed through a 0.45µm filter in duplicate and subsequently the filter is placed on culture medium so that bacteria trapped on the filter paper grow and can be counted as individual colonies, indicating the number of bacteria present in the sample. A modification of the membrane filtration technique could be used to facilitate preferential selection of antimicrobial resistant organisms, the modification being the addition of antimicrobial agents to the agar, and the splitting of the sample into two portions, so that all bacteria present in the portion are enumerated in one portion and bacteria resistant to that antimicrobial agent which grow on the agar with antimicrobial agents and can be enumerated in the second portion.

This method as described does not differentiate between particular species, therefore in order to confirm identify and antimicrobial susceptibility of the species present a representative number of colonies e.g. 5 or 10 are selected for identification. This may be facilitated by initial plating onto differential agar. This modification of the membrane filtration technique would not allow the proportion of sensitive to resistant organisms present in a sample to be determined. Confirmatory testing to establish antimicrobial susceptibility/resistance can be performed using standardized methods such as those of the Clinical Laboratory Standards Institute (CLSI).

An alternative approach involves making up media with individual antibiotics, plating the environmental sample on the medium and incubating the plate to see if any bacteria grow. This method involves preparing media and preparing solutions of the antimicrobials of interest to the desired concentration and pouring the plates. Agar plates prepared in this way containing antimicrobial agents are only useful for 5-7days after preparation; therefore stocks of plates could not be stored for long periods. Also, preparing and pouring plates in this way for large volume use is very time consuming and laborious. Only a small portion (<100µl) of a liquid sample could be examined using this method. Again in order to confirm resistance a representative number of colonies e.g. 5 or 10 are selected and tested for resistance to a panel of antimicrobial agents by a standard method.

The methods described above of determining the presence of antibiotic resistant organisms in a sample and the proportion of antibiotic resistant organisms compared to total numbers of organisms present are time consuming and laborious. None of the methods described facilitate rapid and accurate examination of the proportion of resistant to sensitive organisms present in a sample. The first method is particularly labour intensive and for practical purposes allows only a representative number of bacteria in the sample to be checked for resistance. The second approach is also undesirable since it requires the preparation of the antimicrobial containing media and only a small portion (<100µl) can be examined. Both are relatively demanding of skilled operatives.

At present, there are no regulatory requirements for routine monitoring of water or effluent for the presence of antimicrobial resistant organisms, however there is growing concern regarding the dissemination of antimicrobial resistant organisms and antimicrobial resistant genes in the environment and it is possible the regulatory agencies will require such monitoring in the future.

It is clear that rapid, efficient, facile and inexpensive methods for determining the proportion of antimicrobial resistant bacteria in water samples and other industrial or medical samples are lacking.

International patent application number WO 00/52196 purports to solve a similar problem. The document describes a method of detecting antimicrobial resistant organisms. The method involves a selective enrichment step which requires the selective enrichment of both resistant and non-resistant cells of the pre-selected microbes followed by a further step of subjecting the enriched sample to another enrichment step in a medium and under conditions that will allow the selective enrichment of resistant cells of the microbe. The presence or absence of resistant cells is then detected by way of immunoassay or biotechnological methods, such as DNA or RNA hybdridisation assays. The method permits the absence/presence of resistant organisms such as e.g. Salmonella to be detected in about 24 hours. However, the methods described are complicated and time consuming and involve a number of pre-enrichment steps. In addition, the method described does not allow the determination of the proportion of antimicrobial sensitive to antimicrobial resistant organisms in the sample.

US patent application 09/998,638 relates to a method and kit for performing concurrent identification testing and antimicrobial susceptibility testing from broth culture. The broth must be incubated for about 4 to 6 hours so as to provide adequate numbers of microorganisms for inoculating a multi-chambered kit plate that contains enriched, differential, selective, differential-selective, single-purpose and susceptibility media. The broth is diluted and the plate is inoculated. The more dilute concentration produces individual colonies of microorganisms that can be used for identification testing through tests such as colony characteristic inspection or analysis for characteristic enzyme presence. This isolation makes an initial isolation step unnecessary. The lower dilution provides inoculation for antimicrobial susceptibility tests and other identification tests. In addition, antimicrobial susceptibilities are shown valid even when several different microorganisms coexist in the same test chamber. The susceptibility tests are carried out using known disk-diffusion and micro-dilution tests. The method is fast for bacteria, providing identification and susceptibility data in 24 hrs. However, the method does not provide a means for assessing the proportion of antimicrobial sensitive or antimicrobial resistant organisms in the sample.

The Most Probable Number (MPN) method is routinely used worldwide in the field of Environmental Microbiology to detect and enumerate coliforms, *E. coli* and *Enterococci* in water samples. The MPN method is not an exact count of the numbers of bacteria present in a given sample, but rather an estimate of the most probable number of bacteria in a particular volume of sample. Traditionally this is performed using three sets of tubes with 3 or 5 tubes per set. The first set generally contains 10ml of double strength broth and each tube in the set is inoculated with 10ml of sample. The second and third sets of tubes contain 10ml of single strength broth and each tube in these sets is inoculated with 1ml and 0.1ml of sample respectively. Following incubation the pattern of growth is compared with a table of statistically determined most probable numbers. The method is useful for the detection of total coliforms, *Escherichia coli,* and *Enterococci* in water samples. A variety of commercial kits have been developed for this purpose including the IDEXX™ Colilert (total coliform and *E. coli)* and Enterolert™ *(Enterococci)* kits. The Colilert kit simultaneously detects coliforms and *E*. *coli* in water, and is based on IDEXX's Defined Substrate Technology^{®} (DST^{®}). The sample turns yellow when total coliforms present metabolise the nutrient indicator, ONPG. When *E. coli* are present they metabolise the nutrient indicator, MUG and the sample fluoresces. The Enterolert kit works on the same principles as the Colilert except that the specific nutrient indicator present fluoresces when metabolised by *Enterococci* present in the sample.

These products could be enhanced for the detection of antimicrobial resistant organisms by the methods of this invention. The addition of antimicrobial agents to the MPN method has not previously been examined as a means of detecting the proportion of antimicrobial resistant bacteria present in water samples.

### Object of the Invention

The object of the present invention is to provide processes that could be used to examine the occurrence of antimicrobial resistant organisms in water and effluent, and other liquidised substances such as a suspension, e.g. food, soil, faeces etc. Another object of the invention is to provide a method that is capable of detecting antimicrobial resistant microbes in a number of samples simultaneously. In particular, it is an object of the present invention to provide a less labour intensive way of determining the approximate number of antibiotic resistant *E. coli* and *Enterococci* present in aqueous samples. A further object is to provide a means to effect such detection, a means of testing that is simpler to perform and is faster than standard methods currently used. It is also desirable that such methods would not require pre-enrichment steps before analysis.

Another object of the invention is to provide a means of ascertaining the proportion of antimicrobial sensitive to antimicrobial resistant organisms that are present in a sample.

A further object of the invention is to provide a convenient kit that can accomplish such testing rapidly.

It is a further object to provide such a kit for use in industries such as the environmental, medical and food industries.

It is a further object of the invention to provide a kit designed to facilitate rapid detection of antimicrobial resistant organisms in urine, blood, sputum, and other biological specimens for use by primary care physicians, patients and the like. The biological specimen may be a swab or the like which is used in monitoring the hospital or other medical environment, particularly for resistant organisms associated with hospital acquired infections such as methicillin resistant *Staphylococcus aureus, Pseudomonas aeruginosa* and *Acinetobacter baumanii.*

It is yet a further object of this invention to provide a method for use in the food industry that can identify the presence of resistant strains of bacteria present in food samples and in all areas of the food production process, from farm to processing plants.

It is yet a further object of this invention to provide a method for use in screening hospital effluent, outflow from a wastewater treatment plant and samples (both treated and untreated) from rural group water supplies.

It is also an object of this invention to provide a convenient method for the testing of water quality in emergency and resource-poor settings.

### Summary of the Invention

According to the present invention a method is provided for determining the presence, approximate number and/or the proportion of antimicrobial resistant organisms in a test sample comprising:
(i) mixing at least a portion of the sample with an antimicrobial agent to give a test sample;
(ii) incubating the test sample together with a culture medium;
(iii) testing for growth of the organism in the incubated test sample and determining the levels of any organisms present in the test sample using a most probable number method.

At least another portion of the sample may be incubated in a culture medium without an antimicrobial to give an antimicrobial-free control sample; the level of any organisms present in the control sample being determined using a most probable number method such that the proportion of antimicrobial resistant organisms in the sample can be determined by comparing the number of organisms in the control sample and the number of organisms in the test sample.

Thus the proportion of antimicrobial resistant organisms in the sample can be measured by comparing the value with that of a control sample, which does not contain antimicrobial agent. The most probable number method may be used to enumerate the number of organisms present. This technique involves adding an appropriate indicator to the sample, and incubating the sample, a change in the indicator indicating the presence of the organism. The sample and indicator may be incubated at an appropriate temperature for 18-24 hours. Two nutrient-indicators, o-nitrophenyl-beta-D-galactopyranoside (ONPG) and 4-methylumbeilliferyl-beta-D-glucuronide (MUG), are metabolized by the coliform enzyme β-galactosidase and the *E. coli* enzyme *β-*glucuronidase, respectively. As coliforms they use β-galactosidase to metabolize ONPG and change it from colorless to yellow. As *E. coli* grows it uses β-glucuronidase to metabolize MUG and create fluorescence. Thus these indicators are suitable for use in identifying *E. coli,* although clearly other indicators would be suitable for use with other organisms.

The incubation temperature may vary with regard to the particular organism under investigation, e.g. for *E. coli* incubation at 35°C is appropriate whereas for *Enterococci* incubation at 42⁰C is required.

The number of positive samples are counted and the MPN of organisms present determined by comparison with a predefined table. A number of commercial MPN kits are available. For example, IDEXX Colilert uses the Defined Substrate Technology^{®} to detect total coliforms and *E. coli.* Other kits include the IDEXX Enterolert™ kit for *Enterococci,* the B2P™ Watercheck™, the B2P™ Coliquick™, the B2P™ RCT-100™, and the B2P™RCT-S™ kits.

The method is equally effective for aqueous samples, such as water, seawater, environmental, effluent and even aqueous biological samples such as blood, urine and sputum. Solid samples such as food, faeces, soil, animal bedding can also be tested. Suitably solid samples are liquidised prior to testing. This could be achieved by vortexing/stomaching in a suitable buffer/water.

Aqueous samples can be prepared by serial dilution so as to give a sample within the working range of the test. This is typically in the order of <2000 MPN/100mls. The working range may be in the order of 1 to 2000 MPN/100m1s, preferably 10 to 1500 MPN/100m1s, more preferably 500 to 1000 MPN/100m1s.

The method may be carried out conveniently by use of a kit of parts that contains items necessary to effect the test. The kit may include incubation trays with and without antimicrobial powder and appropriate substrate media. The panel of antimicrobial agents chosen would depend on the antimicrobial resistance one was interested in detecting. In one embodiment a kit to identify MRSA might include oxacillin, methicillin, ciprofloxacin, trimethoprim -sulfamethoxazole, clindamycin and tetracycline, although other combinations of antimicrobial agents could also be used because of variable resistance patterns with MRSA. Such a panel would facilitate detection of the presence of MRSA, and could also assist in determination of whether it was community or hospital acquired, due to known difference in susceptibility patterns of the 2 groups. The advantage of this invention is that kits could be made specific for detection of particular antimicrobial resistant organisms, such as MRSA, or could be more general, in which a broad panel of agents could be included. It would thus be possible to generate kits with different panels of antimicrobial agents.

As mentioned above the indicators, o-nitrophenyl-beta-D-galactopyranoside (ONPG) and 4-methylumbeilliferyl-beta-D-glucuronide (MUG) can be used Conforms use *β-*galactosidase to metabolize ONPG and change it from colorless to yellow. As *E*. *coli* grows it uses β-glucuronidase to metabolize MUG and create fluorescence. Any characteristic of an organism that differentiates it from others could be used. There are a number of chromogenic agars on the market currently, which differentiate organisms by colours based on the interaction of an enzyme produced by the microorganism with substrates in the medium. Other characteristics that could be taken advantage of and used as indicators include lactose fermentation and the production of hydrogen sulphide as well as other enzyme activities etc.

This method allows rapid determination of the presence of antimicrobial resistant organisms in a sample. Previous methods would involve isolation and confirmation of the organism prior to antimicrobial susceptibility testing which could take up to 4 days. This method can confirm the presence of antimicrobial resistant organisms in a sample within 24 hours. Another advantage is that the proportion of resistant to susceptible organisms present can be accurately determined. As outlined above other methods for examination of the proportion of resistant organisms present means that only a small proportion of the sample is examined, and/or only a representative number of organisms isolated are tested.

### Brief Description of the Drawings

Fig. 1: MPN testing of effluent diluted 1:100,000. Yellow wells in Quanti-Tray 2000 are positive for presence of coliforms.
Fig. 2: MPN testing of effluent diluted 1:100,000.. Far left Quanti-Tray 2000; sample with no antibiotic added. Middle Quanti-Tray 2000 contains Ampicillin at 4µg/ml. Far right Quanti-Tray 2000 contains Ampicillin at 32µg/ml. Yellow wells positive for presence of coliforms in all cases.
Fig 3: Quanti-tray 2000, sterile syringe and needle, Chromogenic agar plate.
Fig. 4: Illustration of how aspirate of liquid media is taken from Qunti-Tray 2000.
Fig. 5: Illustration of how aspirate is applied to a UTI Chromogenic agar plate.
Fig 6: Incidence of detection of an antimicrobial resistant organism from effluent samples using the membrane filtration method.
Fig 7: Incidence of detection of an antimicrobial resistant organism from effluent samples using this invention.

Table 1: Accuracy of the invention for detection of resistant organisms if present using samples with known concentrations of sensitive and resistant organisms.
Table 2: Sample of results showing types of water samples that have been tested using the invention. Using the invention in routine practise has allowed for the enumeration of the proportion of antimicrobial resistant total coliforms and *E.coli* present in samples.
Table 3: Sample of disk diffusion susceptibility test results for *E. coli* isolated from various water samples. Resistant results are highlighted in orange
Table 4: Sample results of invention applied to testing seawater.

### Detailed Description of the Invention

### Principle

The principle of the invention is based on a modification of the MPN test. The modification to the normal procedure used in common MPN microbe testing kits effects the method of the invention. The modification is based on an assay carried out by pouring the aqueous sample into a container divided into a large number of individual testing compartments. The container can optionally contain dehydrated components that are reconstituted by the addition of the sample, such that when reconstituted each compartment contains media for growth and growth indicator dyes, which indicate growth of particular microbes. Each well could contain more than one indicator, depending on how many species /groups of organisms were to be detected with the method. When detecting total coliforms and *E. coli,* the substrate added to the water sample contains media for growth and 2 indicators - 1 for detection of coliforms present (ONPG) and one for specific detection of *E*. *coli* (MUG) The compartment is sealed and subsequently incubated, Whether to dilute or not depends on the type of sample. Suitably effluent is diluted 1:100,000; seawater is diluted. 1:10; wafers (other than seawater) are tested neat, although other dilutions would be suitable. Following incubation and depending on the nature of the indicator dyes, when the compartment is examined, colour changes or fluoresence indicate presence of particular organisms. For example in the case of detection of total coliforms present, and when ONPG is the indictor used, in normal light the number of compartments that appear yellow in the container are counted. In the case of detection of *E. coli* present the number of compartments that fluoresce as MUG is metabolised, under UV light arc counted.

By reference to a table developed by the American Public Health association which is a standard table referred to for all MPN methods, this can be accessed in the *Standard Methods for the Examination of Water and Wastewater,* latest edition, 21^{st} Ed., based on probability statistics, the number of one colour of compartment indicates the number of coliforms and the number of the other colour/fluoresence compartments indicates the number of *E. coli* or *Enterococci,* depending on what test and indicator dyes are used. The distinction between total coliforms and *E. coli* is important, as the presence of *E*. *coli* indicates faecal contamination.

In order to get an indication of the proportion of sensitive to resistant organisms present in the aqueous samples, the MPN test is performed concurrently in the presence and absence of antimicrobial agents. This involves mixing a portion (100ml) the sample for analysis with the antimicrobial agent(s) under investigation, and performing the assay, thus allowing identification of the levels of antibiotic resistant organism present in the sample. Alongside this test, a portion (100ml) of the sample is tested in the absence of antimicrobial agents, thereby allowing the total numbers of organisms present to be estimated.

Analysis of combined results of these assays indicate how many organisms are present and what proportion are resistant to antimicrobial agents.

As a result of the EU bathing directive, there is an extensive amount of routine testing of sea water throughout Europe each year. However the current methods routinely used for testing bathing water are slower and vastly more time consuming. The high salt content of seawater interferes with the specificity of the indicator system, but this problem can be overcome in the present invention by diluting the sea water for example 1 in 10 in distilled water, and carrying out the assay of sample versus control in the described manner.

### Example

The invention can be carried out as follows.
- 1000ml of sample is collected.
- This is split into 95-100ml quantities
- One aliquot is tested in the absence of antimicrobial agents
- Antimicrobial agent(s) is added to the aliquots of sample. A single antimicrobial agent is added to a single aliquot of sample.
- The antimicrobial agent is added to achieve the required final concentration in the water sample, e.g. if a final concentration of 4mg/L is required, 4mls of 10mg/L of antimicrobial agent is added to 96mls of sample.
- The following antimicrobial agents were used at the following final concentrations: ampicillin (4µg/ml and 32µg/ml), cefotaxime (.2 µg/ml and 32µg/ml), ciprofloxacin (0.25 µg/ml and 4µg/ml), cefoxitin (2µg/ml and 32µg/ml) and vancomycin (.4 µg/ml and 12.8 µg/ml), but the invention could adapted to include any antimicrobial agent at any required final concentration.
- Samples were then processed using the Colilert ™ and Enterolert ™ systems as per the manufacturers instructions.

Solid samples have to be liquidised prior to testing. This could be achieved by vortexing/stomaching in a suitable buffer/water. Sputum could be prepared by the addition of a liquefying agent such as Sputasol. This renders the sputum more malleable for further manipulation and testing.

### Validation of method

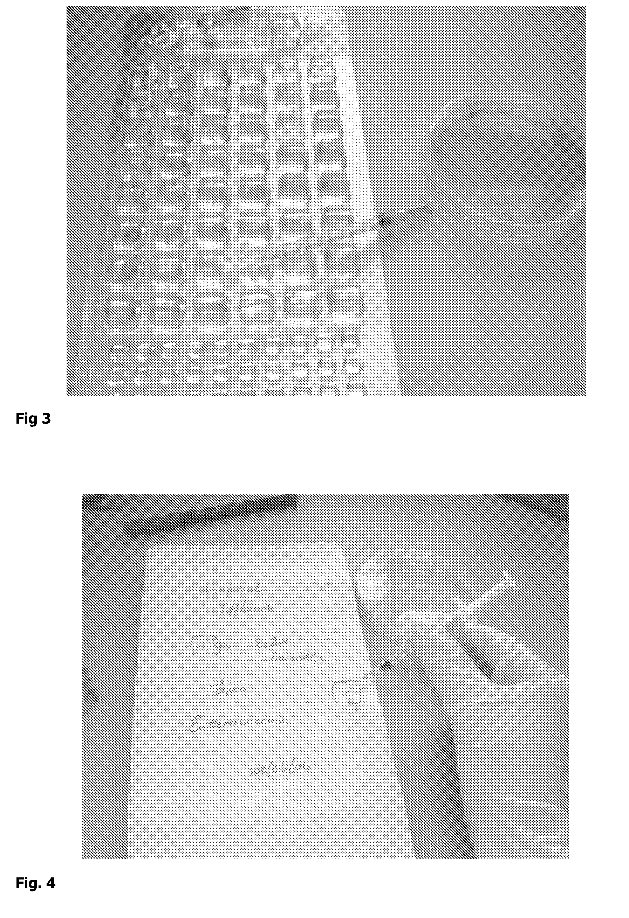

(a) Sensitivity of method - ability to accurately detect the presence of resistant organisms, and the proportion of sensitive to resistant organisms present:
   A sensitive *E.coli* ATCC 25922 and clinically isolated *E. coli* isolates, with known resistance to cefotaxime (MIC ≥16µg/ml {minimum inhibitory concentration (MIC) is the lowest concentration of antimicrobial required to inhibit growth of the bacterium}) and Ampicillin, Cefoxitin and Ciprofloxacin (MIC= >32µg/ml) were used. A suspension of the sensitive and resistant organisms were prepared and adjusted using a VITEK colorimeter to be equal in turbidity to a 0.5 McFarland standard. A 0.5 McFarland suspension is equivalent to 1 to 2 x 10⁸ colony forming units/ml (CFU/ml), according to Clinical Laboratory Standards Institute (CLSI) guidelines. Serial dilutions of the samples were performed in order to reach a readable level of bacteria for the method i.e., <2000 MPN/100mls. The MPN method is not an exact count of the numbers of bacteria present in a given sample, but rather a statistical estimation of the most probable number of bacteria in a particular volume of sample. MPN is usually expressed per 100ml of sample. CFU is a bacterial colony presumed to have originated from a single bacterium present in the sample. Approximately 1 to 2 x 10² CFU/ml of each bacterium were added to sterile water, to a final volume of 100mls and tested using the IDEXX Colilert system, both with and without the addition of cefotaxime (2µg/ml, 32µg/ml), ampicillin (4µg/ml, 32µg/ml), ciprofloxacin (0.25µg/ml, 4µg/ml) and cefoxitin (32µg/ml). Three suspensions were prepared in the absence of antimicrobial agents (Control 1 = resistant organisms only, Control 2 = sensitive and resistant organisms together, Control 3 = sensitive organisms only). Control 4 contained sensitive and resistant organisms and antimicrobial agent/. Results for this validation carried out using cefotaxime (2mg/L) are shown in Table 1. In each instance the approximate number of sensitive versus resistant organisms was retrieved. The results show that in the absence of antimicrobial agent the approximate number of organisms added is retrieved. The results also show that upon addition of the antimicrobial agent (in the example data given cefotaxime (2mg/L)) that only the resistant organisms present in the sample are retrieved. This demonstrates the specificity of the invention for the preferential detection of antimicrobial resistant organisms if present in a sample. The results also demonstrate the ability of the invention to determine the proportion of sensitive to resistant organisms present in the sample.
(b) Confirmation of antimicrobial resistance preselected for:
   - In all testing carried out to date two wells from any tray positive for *E. coli* or *Enterococci* (indicator fluorescence in both cases) were aspirated using a sterile syringe (Figs 3 to 5), plated onto a chromogenic agar plate. This confirms the identity of the species.
   - A single colony was then sub cultured onto a Muller Hinton agar plate and incubated at 35°C for 18 hours.
   - Antimicrobial susceptibility testing to a panel of 15 antimicrobial agents was carried out in accordance Clinical Laboratory Standard Institute (CLSI) disk diffusion methods.
   - In all cases, the resistance selected for using the addition of antimicrobial agents in combination with the MPN method was confirmed by disk diffusion testing (Table3). This data indicates that in all cases all *E. coli* isolated were resistant to one or more of the antimicrobial agents screened for.

### Sampling

A variety of water samples have been examined using this modification of the MPN method. These include samples of hospital effluent, outflow from a wastewater treatment plant, source and treated samples from rural group water supplies, and bathing seawater samples.

### Routine use of method

100mls of each sample was tested using the Colilert ™ and Enterolert ™ systems. Group water scheme samples were tested neat. Other samples required dilution in sterile water. Sea water was diluted 1:10, wastewater treatment outflow was diluted 1:100, and hospital effluent was diluted 1:100,000.

Each water/effluent sample was tested in the presence and absence of the following antimicrobial agents at the following final concentrations: cefotaxime 2µg/ml, 32µg/ml, ciprofloxacin 0.25 µg/ml, 4µg/ml, ampicillin 4µg/ml, 32µg/ml, cefoxitin 32µg/ml, streptomycin 8µg/ml, tetracycline 4µg/ml, vancomycin 256µg/ml.

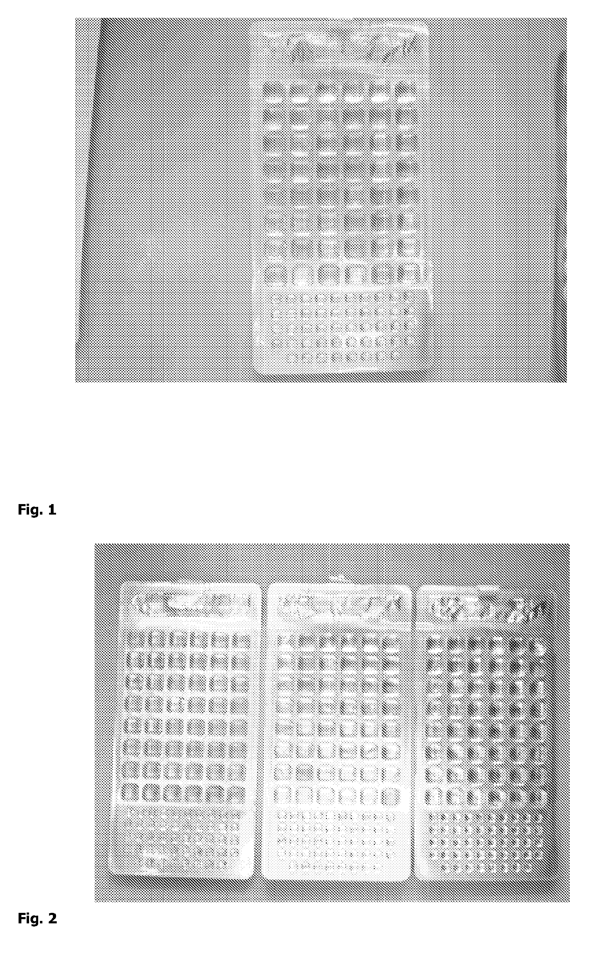

For each sample in which *E. coli* and/or *Enterococci* were identified, 2 positive wells per tray were aspirated and cultured on Chromogenic UTI agar alone for *E. coli* and also onto Slanetz + Bartley + Vancomycin agar (LIP) to confirm presence of vancomycin resistant *Enterococci* (See Figs. 1 to 5) These isolates were then stored at -70°C for further investigation.

### Validation of sensitivity of methods

The antimicrobial susceptibility of each *E.coli* and *Enterococci* isolate obtained was determined in accordance with standard Clinical Laboratory Standards Institute (CLSI) disk diffusion methods. A suspension of the test organism was prepared and adjusted using a VITEK colorimeter to be equal in turbidity to a 0.5 McFarland standard. This was then swabbed onto the surface of a Mueller Hinton agar plate. Disks impregnated with antimicrobial agents at a certain concentration (see below) were placed onto the surface of the swabbed plate. These were then incubated overnight at 35°C. Zones of inhibition were recorded and compared with zone diameter interpretive standards as defined by CLSI. For *E.coli* the following antimicrobial agents were tested: ampicillin (10µg/ml), cefpodoxime (10µg/ml), cefpodoxime/clavulanic acid, ceftazidime (30µg/ml), cefoxitin (30µg/ml), ciprofloxacin (5µg/ml), nalidixic acid (30µg/ml), gentamycin (10µg/ml), kanamycin (30µg/ml), sulphonamides (300µg/ml), trimethoprim (5µg/ml), tetracycline (30µg/ml) and minocycline (30µg/ml). For Enterococci, ampicillin 10ug, gentamycin 120ug, vancomycin 30ug, linezolid (15µg/ml), quinupristin/dalfopristin (30µg/ml) and teicoplanin (30µg/ml) were tested.

In all instances the resistance identified using the MPN method was confirmed by antimicrobial susceptibility testing (Table 3). Antimicrobial agents of different class and concentration were added to the sample, and following incubation, positive wells were sub cultured to confirm antimicrobial sensitivity. Confirmation tests indicated 100% specificity with regard to the particular antimicrobial agent under investigation.

### Comparison of Modified MPN method with other selection methods

Prior to developing the Colilert ™ / antibiotic method, membrane filtration was the method used to quantify and detect resistant organisms in routine samples. A water/effluent sample would be collected and passed through a membrane filtration manifold and the membrane placed directly onto Chromogenic ™ UTI agar.

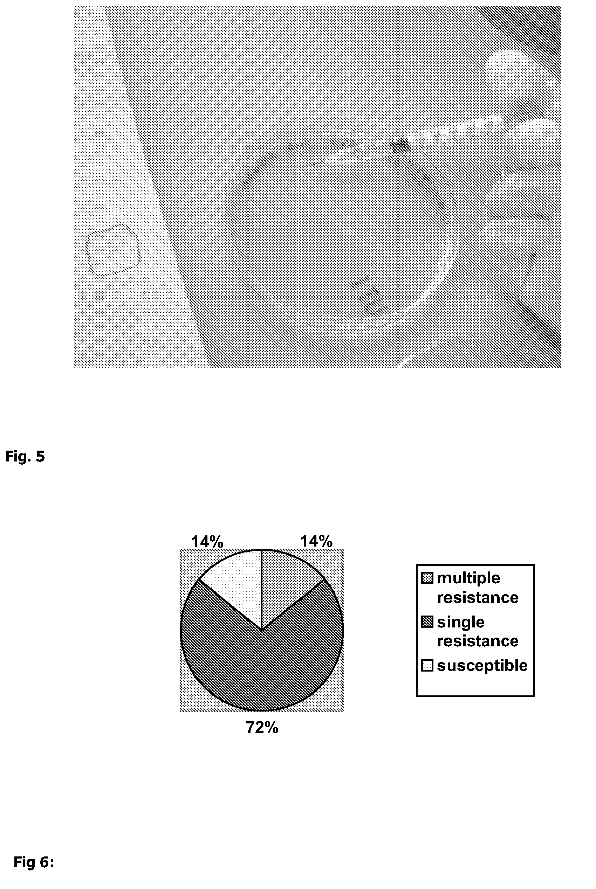

From this plate 10 *E.coli* and 10 *Enterococci* were selected at random and antimicrobial susceptibility testing performed in accordance with CLSI disk diffusion criteria. The main disadvantage to this method is that there is no preferential selection for antimicrobial resistant organisms, and no information on the proportion of resistant to sensitive organisms present is attained. Out of 20 randomly selected *E.coli* isolated using the direct plating technique and tested for antimicrobial susceptibility using CLSI guidelines, 6 (30%) were resistant to one or more antibiotics (Fig. 6).

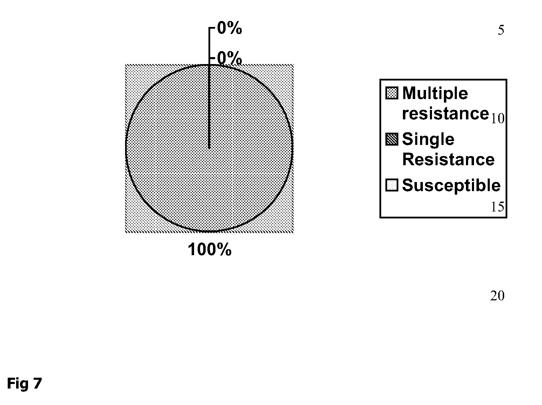

The modified MPN method increased the probability of detecting antimicrobial resistant bacteria and also offered the advantage of being able to determine the proportion of sensitive to resistant organisms present in the sample. Out of 20 *E.coli's* isolated using the modified MPN method and CLSI testing, 100% of isolates were resistant to 2 or more antibiotics tested (Fig. 7) The following antimicrobial agents were tested: ampicillin, chloramphenicol, streptomycin, sulphonamides, tetracycline, trimethoprim, naladixic acid, kanamycin, ciprofloxacin, cefpodoxime, cefpodoxime/clavulanic acid, ceftazidime, cefoxitin, gentamicin, minocycline, and cefotaxime (Table 3).

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

**Table 1**

| Sample # | Sample: | Bacterial added | Antibiotic added: | Coliform MPN/100mls | | E.coli MPN/100mls | |
|---|---|---|---|---|---|---|---|
| 1 | Control | 1ml Resistant (10²) | no antibiotic | 365.4 | **3.65** | 365.4 | **3.65 MPN/ml** |
| 2 | Control | 2mls Sensitive + 1ml Resistant (10²) | no antibiotic | 816.4 | **8.164** | 816.4 | **8.164 MPN/ml** |
| 3 | Control | 2 mls Sensitive (10²) | no antibiotic | 344.8 | **3.44** | 344.8 | **3.44 MPN/ml** |
| 4 | Control | 2mls Sensitive + 1ml Resistant (10²) | **Cefotaxime 2 µg/ml** | 3.654 | **3.654** | 3.654 | **3.654 MPN/ml** |

**TABLE2:**

| **Sample type:** | **E.coli: MPN/100mls** | **CIP* 0.25µg/ml** | **4µg/ml** | **CTX* 2µg/ml** | **32µg/ml** | **AMP* 4µg/ml** | **32µg/ml** | **FOX* 32µg/ml** | **TET* 4µg/ml** | **STREP 8µg/ml** |
|---|---|---|---|---|---|---|---|---|---|---|
| | | **MPN/100mls of sample** | | | | | | | | |
| Hospital effluent_{site 1} | **4.7 x 10⁶** | /* | **6.3 x 10⁵** | **5.2 x 10⁴** | **7 x 10³** | / | **1.4 x 10⁶** | **3.1 x 10⁵** | / | / |
| Hospital effluent _{site 2} | **8.4 x 10⁵** | / | <100,000** | / | <100,000 | / | **5.2 x 10⁵** | <100,000 | / | / |
| Sea water | **5.3 x 10²** | **100** | / | < 10 | / | **537** | / | < 100 | / | / |
| Treatment Plant Outflow | **1.18 x 10⁴** | < 100 | / | / | **86** | / | **1,100** | <100 | / | / |
| Group water scheme K | Source: **78** | <1 | / | <1 | / | **11** | / | <1 | **4.1** | **62.4** |
| | Treated: **178.35** | **1** | / | <1 | / | **22.2** | / | <1 | **3.1** | **62.4** |
| Group water scheme C | Source: **26.4** | <1 | / | <1 | / | **1** | / | <1 | 1 | **32.4** |
| | Treated: **31.05** | <1 | / | <1 | / | **3.1** | / | <1 | <1 | **19.2** |
| Group water scheme M | Source: **22.2** | <1 | / | <1 | / | **2** | / | <1 | **3.1** | **13.5** |
| | Treated: <1 | <1 | / | <1 | / | <1 | / | <1 | <1 | <1 |

| | | | | | | | | | | |
|---|---|---|---|---|---|---|---|---|---|---|
| Note: / = **samples not tested with antibiotic concentration listed, due to type of sample, e.g. effluent is tested at a higher concentration that drinking water, as it is more contaminated and more likely to have high levels of resistant bacteria**. [CIP = Ciprofloxacin; CTX = Cefotaximr; AMP = Ampicillin, FOX = Cefoxitin; TET = Tetracycline; STREP = Streptomycin **< = In a neat sample, no positive wells means a result of <1. When the dilution factor is taken into account this result must also be factored in. e.g. for effluent a negative result is actually <100,000 MPN/100mls. | | | | | | | | | | |

**Table 3:**

| **Location** | **Specimen number** | **Specimen date** | **Organism** | **AMP** | **CHL** | **STR** | **SSS** | **TCY** | **TMP** | **NAL** | **KAN** | **CIP** | **CPD** | **CDC** | **CAZ** | **FOX** | **GEN** | **MNO** | **CTX** |
|---|---|---|---|---|---|---|---|---|---|---|---|---|---|---|---|---|---|---|---|
| After | s-0359 | 05/02/2007 | eco | 6 | 18 | 12 | 6 | 18 | 27 | 19 | 16 | 24 | 21 | 21 | 29 | 26 | 19 | 20 | 33 |
| After | s-0361 | 05/02/2007 | eco | 6 | 6 | 10 | 6 | 6 | 6 | 6 | 20 | 6 | 18 | 18 | 28 | 9 | 9 | 6 | 29 |
| Before | s-0250 | 28/11/2006 | eco | 6 | 18 | 11 | 6 | 21 | 24 | 21 | 6 | 31 | 23 | 23 | 25 | 24 | 20 | 21 | 30 |
| Before | s-0291 | 24/01/2007 | eco | 6 | 17 | 6 | 6 | 20 | 6 | 26 | 21 | 31 | 27 | 28 | 31 | 25 | 20 | 21 | 32 |
| Before | s-0330 | 01/02/2007 | eco | 6 | 22 | 6 | 6 | 6 | 6 | 25 | 24 | 33 | 6 | 6 | 19 | 10 | 24 | 11 | 26 |
| Before | s-0360 | 05/02/2007 | eco | 6 | 21 | 15 | 24 | 6 | 27 | 6 | 10 | 6 | 24 | 24 | 28 | 20 | 6 | 18 | 30 |
| Before | s-0362 | 05/02/2007 | eco | 6 | 19 | 16 | 20 | 6 | 29 | 6 | 21 | 31 | 27 | 27 | 32 | 24 | 20 | 17 | 34 |
| CSW | s-0283 | 24/01/2007 | eco | 6 | 6 | 6 | 6 | 6 | 26 | 25 | 24 | 32 | 28 | 27 | 30 | 27 | 21 | 9 | 34 |
| CSW | s-0404 | 24/01/2007 | eco | 6 | 20 | 6 | 6 | 6 | 6 | 6 | 21 | 6 | 23 | 24 | 26 | 19 | 19 | 10 | 29 |
| CSW | s-0405 | 24/01/2007 | eco | 6 | 6 | 6 | 6 | 6 | 6 | 21 | 6 | 31 | 24 | 24 | 28 | 21 | 18 | 17 | 30 |
| CSW | s-0406 | 24/01/2007 | eco | 13 | 18 | 15 | 6 | 6 | 6 | 20 | 20 | 26 | 24 | 22 | 27 | 19 | 18 | 15 | 30 |
| CSW | s-0417 | 01/02/2007 | eco | 6 | 21 | 6 | 6 | 24 | 29 | 26 | 22 | 32 | 28 | 29 | 33 | 29 | 22 | 23 | 32 |
| K | s-0156 | 04/10/2006 | eco | 6 | 21 | 10 | 24 | 6 | 25 | 22 | 20 | 30 | 24 | 24 | 24 | 23 | 20 | 10 | 28 |
| K | s-0157 | 04/10/2006 | eco | 6 | 23 | 6 | 6 | 6 | 26 | 24 | 6 | 31 | 22 | 22 | 25 | 19 | 20 | 18 | 27 |
| K | s-0169 | 18/10/2006 | eco | 6 | 6 | 6 | 6 | 6 | 6 | 22 | 6 | 29 | 23 | 22 | 26 | 20 | 19 | 11 | 30 |
| K | s-0169 | 18/10/2006 | eco | 6 | 6 | 6 | 7 | 6 | 6 | 21 | 6 | 31 | 22 | 22 | 26 | 20 | 20 | 10 | 30 |
| K | s-0425 | 31/01/2007 | eco | 6 | 22 | 6 | 6 | 6 | 6 | 23 | 22 | 32 | 26 | 26 | 29 | 23 | 20 | 16 | 36 |
| K | s-0426 | 31/01/2007 | eco | 6 | 15 | 6 | 6 | 6 | 6 | 23 | 20 | 30 | 25 | 25 | 28 | 24 | 20 | 15 | 33 |
| MIO | s-0108 | 08/09/2006 | eco | 6 | 22 | 16 | 24 | 23 | 24 | 22 | 18 | 30 | 6 | 18 | 23 | 21 | 11 | 23 | 9 |
| MIO | s-0111 | 15/09/2006 | eco | 6 | 23 | 7 | 6 | 6 | 6 | 6 | 19 | 7 | 23 | 24 | 26 | 21 | 20 | 12 | 29 |

**Table 4:**

| **Sample** | **Antibiotic** | **Dilution** | **Total Coliform MPN/100ml** | **Calculated with dilution factor** | **E. coli MPN /100ml** | **Calculated with dilution factor** |
|---|---|---|---|---|---|---|
| Sea water | **no antibiotic** | 1/10 | 0 | **<10** | 0 | **<10** |
| Sea water | Cefotaxime 2µg/ml | 1/10 | 0 | **<10** | 0 | **<10** |
| Sea water | Ampicillin 4µg/ml | 1/10 | 1 | **10** | 0 | **<10** |
| Sea water | Cefoxitin 32µg/ml | 1/10 | 0 | **<10** | 0 | **<10** |
| Sea water | Ciprofloxacin 0.25µg/ml | 1/10 | 0 | **<10** | 0 | **<10** |
| Sea water | **no antibiotic** | 1/10 | 6.3 | **63** | 0 | **<10** |
| Sea water | Cefotaxime 2µg/ml | 1/10 | 0 | **<10** | 0 | **<10** |
| Sea water | Ampicillin 4µg/ml | 1/10 | 0 | **<10** | 0 | **<10** |
| Sea water | Cefoxitin 32µg/ml | 1/10 | 0 | **<10** | 0 | <10 |
| Sea water | Ciprofloxacin 0.25µg/ml | 1/10 | 0 | **<10** | 0 | <10 |
| Sea water | **no antibiotic** | 1/10 | 2 | 20 | 0 | <10 |
| Sea water | Cefotaxime 2µg/ml | 1 /10 | 0 | <10 | 0 | <10 |
| Sea water | Ampicillin 4µg/ml | 1/10 | 7.5 | 75 | 1 | 10 |
| Sea water | Cefoxitin 32µg/ml | 1/10 | 0 | <10 | | <10 |
| Sea water | Ciprofloxacin 0.25µg/ml | 1/10 | 0 | <10 | 0 | <10 |
| Sea water | **no antibiotic** | 1/10 | 109.1 | **1,091** | 101.3 | **1,013** |
| Sea water | Cefotaxime 2µg/ml | 1/10 | 0 | **<10** | 0 | **<10** |
| Sea water | Ampicillin 4µg/ml | 1/10 | 101.3 | **1,013** | 83.1 | **831** |
| Sea water | Cefoxitin 32µg/ml | 1/10 | 0 | **<10** | 0 | **<10** |
| Sea water | Clprofloxacin 0.25µg/ml | 1/10 | 0 | **<10** | 0 | **<10** |
| Sea water | **no antibiotic** | 1/10 | 95.9 | **9.6 x 10²** | 52.9 | **5.3 x 10²** |
| Sea water | Cefotaxime 2µg/ml | 1/10 | 0 | **<10** | 0 | **<10** |
| Sea water | Ampicillin 4µg/ml | 1/10 | 70.3 | **703** | 53.7 | **537** |
| Sea water | Cefoxitin 32µg/ml | 1/10 | 43.2 | **4.3 x 10³** | 0 | **<100** |
| Sea water | Ciprofloxacin 0.25µg/ml | 1/10 | 4.1 | **4.1 x 10²** | 1 | **100** |

## Claims

1. A method is provided for determining the presence and/or the proportion of antimicrobial resistant organisms in a sample comprising:
(i) mixing at least a portion of the sample with an antimicrobial agent to give a test sample;
(ii) incubating the test sample together with a culture medium;
(iii) testing for growth of the organism in the incubated test sample and determining the levels of any organisms present in the test sample using a most probable number method.

2. The method as claimed in claim 1, wherein the sample is diluted before incubation.

3. The method as claimed in claim 1 or claim 2, wherein at least a portion of the sample is incubated in a culture medium without an antimicrobial to give a control antimicrobial-free sample; the level of any organisms present in the control sample being determined using a most probable number method such that the proportion of antimicrobial resistant organisms in the sample can be determined by comparing the number of organisms in the control sample and the number of organisms in the test sample.

4. The method according to any preceding claim wherein the test or control sample is incubated in a medium containing an indicator which indicates growth of an organism.

5. The method as claimed in any preceding claim wherein the sample is selected from the group comprising an aqueous sample, a solid sample, a food sample, an environmental sample or a biological sample.

6. The method according to claim 5, wherein the aqueous sample is selected from the group comprising water, seawater and effluent.

7. The method according to claim 5, wherein the solid sample is liquidised in a buffer or water prior to incubation.

8. The method according to claim 5, wherein the biological sample is sputum faeces, urine or blood and the sample is mixed with an liquefying agent such as Sputasol.

9. The method as claimed in claim 6, wherein a 1:10 dilution of the aqueous sample is carried out.
